# EUROPEAN PATENT APPLICATION

(11) **EP 4 623 958 A1**
(43) Date of publication of application: **01.10.2025**
(21) Application number: 23922812.5
(22) Date of filing: 19.06.2023
(51) Int. Cl.: A61M 5/28, A61M 5/32, A61J 1/20

(54) **SYRINGE BARREL, SYRINGE, DRUG PREPARATION SET, AND METHOD FOR MANUFACTURING SYRINGE BARREL**

(30) Priority: 14.02.2023 JP 2023021200
(71) Applicant: Terumo Kabushiki Kaisha, Tokyo 151-0072 (JP)
(72) Inventor: OKIHARA, Hitoshi, Fujinomiya-shi, Shizuoka 418-0004 (JP)
(74) Representative: KIPA AB
(86) International application number: PCT/JP2023/022599
(87) International publication number: WO 2024/171478

(57) **Abstract**

A syringe barrel 1 includes a connection blocking member 30 that blocks the connection of another medical tool (such as an injection needle) to a tip portion 13 and/or a collar portion 14, and an inner surface of the collar portion 14 including a barrel-side threaded portion 15 and an outer surface of a cylindrical main body portion 31 including a blocking member-side threaded portion 32 include a rotation restricting engagement portion 16 that is partially engaged when the barrel-side threaded portion 15 and the blocking member-side threaded portion 32 are screwed together and restricts rotation of the connection blocking member 30 in the collar portion 14 in the screwing release direction.

## Description

### Technical Field

The present invention relates to a syringe barrel including a connection blocking member that blocks connection of another medical tool (such as an injection needle) to a tip portion and/or a collar portion, a syringe, a drug preparation set, and a method for manufacturing a syringe barrel.

### Background Art

Conventionally, a plurality of types of drugs stored in separate medical instruments (syringes, vials, and the like) is mixed before administration (injection), and the mixed drug is administered (injection). As a medical instrument used in such a method, for example, there is a medical instrument including a syringe storing one drug and a vial storing other drugs, in which both are connected using a connection medical tool to mix a plurality of types of drugs, an injection needle is attached to the syringe storing the mixed drug, and the drug is administered (injected) to a patient.

Meanwhile, the applicants of the present application disclose a syringe set, a syringe, and a set for the purpose of preventing erroneous administration due to forgetting to mix drugs before administration in Patent Literature 1 (WO2021-182370). Specifically, in the syringe set disclosed in Patent Literature 1, the first connection port of the first syringe storing the first drug includes an injection needle attachment structure to which an injection needle can be attached and a blocking member that blocks attachment of the injection needle to the injection needle attachment structure, and when the second connection port of the drug container storing the second drug is connected to the first connection port, the blocking member is connected to the second connection port side, and the second connection port to which the blocking member is connected is detached from the first connection port, so that the blocking member is detached from the first connection port and the injection needle can be attached to the injection needle attachment structure. According to such a syringe set, since the injection needle is not attached if the mixing is forgotten, it is possible to prevent erroneous administration due to the mixing being forgotten.

### Citation List

### Patent Literature

Patent Literature 1: WO2021-182370 (US2022-354747A, EP4115928A)

### Summary of Invention

### Technical Problem

In the syringe set of Patent Literature 1, the blocking member is stored inside the luer lock portion of the syringe with a gap or with slight contact to have frictional resistance. Therefore, there is a possibility that the blocking member unintentionally falls off due to vibration at the time of transportation, operation at the time of use (such as turning the syringe distal end downward after opening the cap), and the like.

In addition, even if the blocking member is slightly in contact with the inner side of the luer lock portion of the syringe and stored with frictional resistance, when heat is applied by a treatment of high-pressure steam sterilization or the like after the attachment step of the blocking member, the frictional resistance may be lost due to thermal deformation, and the blocking member may unintentionally fall off from the syringe.

Therefore, an object of the present invention is to provide a syringe barrel including a connection blocking member that blocks connection of another medical tool (such as an injection needle) to a tip portion and/or a collar portion, a syringe, and a drug preparation set, and a method for manufacturing a syringe barrel, in which the connection blocking member is less likely to fall off.

### Solution to Problem

The above object is achieved by the following.
(1) A syringe barrel including: a barrel having a barrel main body capable of storing a drug, a hollow tip portion provided at a distal portion of the barrel main body and a collar portion covering the tip portion and including a barrel-side threaded portion on an inner surface; and a connection blocking member disposed between the tip portion and the collar portion of the barrel and blocking connection of another medical tool to the tip portion and/or the collar portion, in which
   the connection blocking member includes a cylindrical main body portion and a blocking member-side threaded portion that is formed on an outer surface of the cylindrical main body portion and is screwable with the barrel-side threaded portion, and
   an inner surface of the collar portion including the barrel-side threaded portion and an outer surface of the cylindrical main body portion including the blocking member-side threaded portion partially engage with each other at a time of screwing the barrel-side threaded portion and the blocking member-side threaded portion, and include a rotation restricting engagement portion that restricts rotation of the connection blocking member in the collar portion in a screwing release direction.
(2) The syringe barrel according to (1), in which the barrel-side threaded portion is a spiral recessed portion, and the blocking member-side threaded portion is a screw rib that is screwable with the spiral recessed portion.
(3) The syringe barrel according to (2), in which the connection blocking member includes a protruding portion provided on an upper surface of the screw rib forming the blocking member-side threaded portion, the spiral recessed portion forming the barrel-side threaded portion includes an inner surface site to be engaged with the protruding portion, and the rotation restricting engagement portion is formed by the protruding portion and the inner surface site of the spiral recessed portion.
(4) The syringe barrel according to (3), in which the inner surface site includes a recessed portion that is engaged with the protruding portion and stores a vertex portion of the protruding portion.
(5) The syringe barrel according to (2), in which the screw rib forming the blocking member-side threaded portion includes a raised portion formed higher than other portions, the spiral recessed portion forming the barrel-side threaded portion includes an inner surface site to be engaged with the raised portion, and the rotation restricting engagement portion is formed by the raised portion and the inner surface site of the spiral recessed portion.
(6) The syringe barrel according to (2), in which the connection blocking member includes the screw rib forming the blocking member-side threaded portion, the spiral recessed portion forming the barrel-side threaded portion includes a low recessed site having a recessed portion depth shallower than other sites, and the rotation restricting engagement portion is formed by the low recessed site and an outer surface of the screw rib.
(7) The syringe barrel according to any one of (1) to (6), in which the syringe barrel includes a distal end annular space formed between an inner surface of a cylindrical distal portion of the connection blocking member and an outer surface of the tip portion of the barrel.
(8) The syringe barrel according to any one of (3) to (5), in which a protrusion amount and/or a width of the protruding portion or the raised portion decreases in a screwing direction of the screw rib of the connection blocking member.
(9) The syringe barrel according to any one of (1) to (8), in which the connection blocking member is formed of a thermoplastic resin harder than a thermoplastic resin forming the barrel.
(10) The syringe barrel according to any one of (1) to (9), in which the connection blocking member is formed of a thermoplastic resin having a thermal deformation temperature higher than a thermoplastic resin forming the barrel.
(11) The syringe barrel according to any one of (2) to (6) and (8), in which in the cylindrical main body portion of the connection blocking member, a portion where the screw rib is formed is a thick portion having a wall thickness larger than other sites.
(12) The syringe barrel according to any one of (1) to (11), in which the cylindrical main body portion of the connection blocking member includes a cutout portion that opens in a distal end direction at a distal end side portion.
(13) The syringe barrel according to any one of (1) to (12), in which the syringe barrel includes a cap that seals the tip portion, the barrel includes a male screw portion provided on an outer surface of the collar portion, and the cap includes a cap main body portion having a female screw portion screwed to the male screw portion of the collar portion, and a sealing member that is supported by the cap main body portion and abuts on a distal portion of the tip portion to seal the tip portion.
   In addition, the object described above is achieved by the following.
(14) A syringe including: the syringe barrel according to any one of (1) to (13); a gasket slidably stored in the syringe barrel; and a plunger attached to or attachable to the gasket.
   In addition, the object described above is achieved by the following.
(15) A drug preparation set including: the syringe barrel according to any one of (1) to (13); and
   a connection medical tool including a connection portion connectable to the tip portion of the syringe barrel, in which
   the syringe barrel includes a distal end annular space formed between an inner surface of a cylindrical distal portion of the connection blocking member and an outer surface of the tip portion of the barrel,
   the connection portion of the connection medical tool includes an inner tube portion capable of entering the distal end annular space of the syringe barrel and an outer tube portion covering the inner tube portion,
   the inner tube portion has an inner surface shape that is liquid-tightly engageable with an outer surface of the tip portion, includes an engagement portion that is engageable with an inner surface of the connection blocking member on an outer surface, and communicates with an inside of the barrel via the tip portion in a connection state between the tip portion of the barrel and the connection medical tool, and
   the syringe barrel or the connection medical tool is rotated in a direction in which screwing between the barrel and the connection blocking member is released in a state in which the engagement portion of the inner tube portion of the connection medical tool and the inner surface of the connection blocking member are engaged, such that the connection blocking member is detached from the barrel together with the connection medical tool, and other medical tools are connectable to the tip portion of the barrel and/or the collar portion.
(16) The drug preparation set according to (15), in which an uneven structure engageable with each other is formed on an inner surface of the connection blocking member and an outer surface of the inner tube portion of the connection medical tool.
(17) The drug preparation set according to (16), in which the uneven structure includes an annular protrusion extending in a circumferential direction.
(18) The drug preparation set according to (16), in which the uneven structure includes a longitudinal rib extending in an axial direction.
(19) The drug preparation set according to (16), in which the uneven structure includes a ratchet structure that rotates the connection blocking member only in a direction in which the connection blocking member is separated from the barrel.
(20) The drug preparation set according to any one of (15) to (19), in which the connection medical tool is a vial adapter.
   In addition, the object described above is achieved by the following.
(21) A method for manufacturing the syringe barrel according to (3) or (4),
   the method including:
   a step of preparing the barrel and the connection blocking member;
   a connection blocking member mounting step of disposing the connection blocking member between the tip portion and the collar portion by screwing, and engaging the protruding portion provided on an upper surface of the screw rib forming the blocking member-side threaded portion with a part of the spiral recessed portion forming the barrel-side threaded portion; and
   a heating step of heating the barrel to which the connection blocking member is mounted by the connection blocking member mounting step, and embedding a vertex site of the protruding portion of the connection blocking member in an inner surface of the spiral recessed portion forming the barrel-side threaded portion by deformation of the barrel due to the heating.
(22) The method for manufacturing the syringe barrel according to (21), in which the heating step is performed by high-pressure steam sterilization.

### Brief Description of Drawings

Fig. 1 is a front view illustrating an example of a syringe barrel of the present invention.
Fig. 2 is a cross-sectional view taken along line A-A of Fig. 1.
Fig. 3 is a cross-sectional view illustrating a barrel constituting the syringe barrel illustrated in Fig. 1.
Fig. 4 is an enlarged cross-sectional view illustrating a distal end portion of a barrel illustrated in Fig. 3.
Fig. 5 is a front view illustrating a connection blocking member constituting the syringe barrel illustrated in Fig. 1.
Fig. 6 is a cross-sectional view taken along line B-B of Fig. 5.
Fig. 7 is a bottom view of a connection blocking member illustrated in Fig. 5.
Fig. 8 is an enlarged view of a portion C in Fig. 7.
Fig. 9 is an enlarged view of a portion D in Fig. 7.
Fig. 10 is an enlarged cross-sectional view illustrating a distal end portion of the syringe barrel illustrated in Fig. 1.
Fig. 11 is an enlarged view of a portion E in Fig. 10.
Fig. 12 is a front view illustrating an example of the syringe of the present invention.
Fig. 13 is a cross-sectional view taken along line F-F in Fig. 12.
Fig. 14 is a front view illustrating an example of a connection medical tool constituting a drug preparation set of the present invention.
Fig. 15 is a plan view of the connection medical tool illustrated in Fig. 14.
Fig. 16 is an enlarged view of a portion G in Fig. 15.
Fig. 17 is a cross-sectional view taken along line H-H in Fig. 15.
Fig. 18 is a cross-sectional view illustrating an example of a vial to which the connection medical tool (vial adapter) illustrated in Fig. 14 can be attached.
Fig. 19 is an explanatory cross-sectional view for explaining a method for using the drug preparation set of the present invention.
Fig. 20 is an explanatory cross-sectional view for explaining a method for using the drug preparation set of the present invention.
Fig. 21 is an explanatory cross-sectional view for explaining a method for using the drug preparation set of the present invention.
Fig. 22 is an enlarged explanatory cross-sectional view taken along line J-J in Fig. 21.
Fig. 23 is an explanatory cross-sectional view for explaining a method for using the drug preparation set of the present invention.
Fig. 24 is a cross-sectional view corresponding to Fig. 11, illustrating another example of the syringe barrel of the present invention.
Fig. 25 is a cross-sectional view corresponding to Fig. 11, illustrating another example of the syringe barrel of the present invention.
Fig. 26 is a cross-sectional view corresponding to Fig. 11, illustrating another example of the syringe barrel of the present invention.
Fig. 27 is a cross-sectional view corresponding to Fig. 11, illustrating another example of the syringe barrel of the present invention.

### Description of Embodiments

A syringe barrel, a syringe, a drug preparation set, and a method for manufacturing a syringe barrel of the present invention will be described with reference to examples illustrated in the drawings.

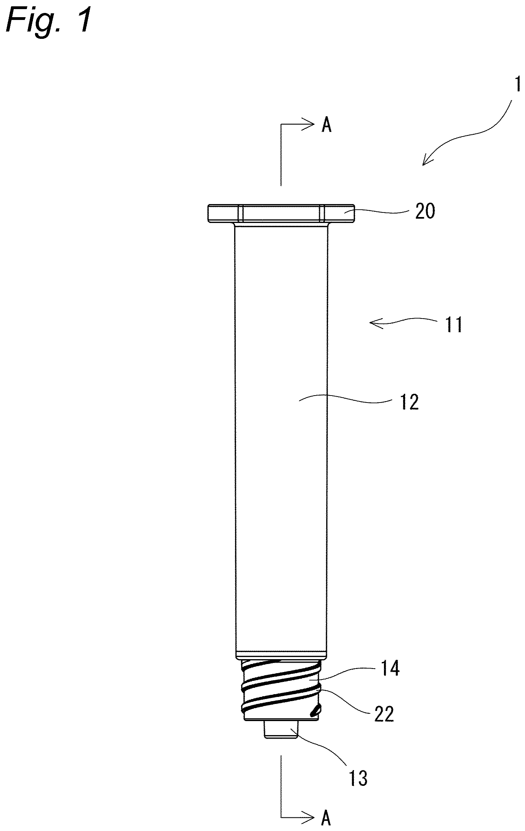

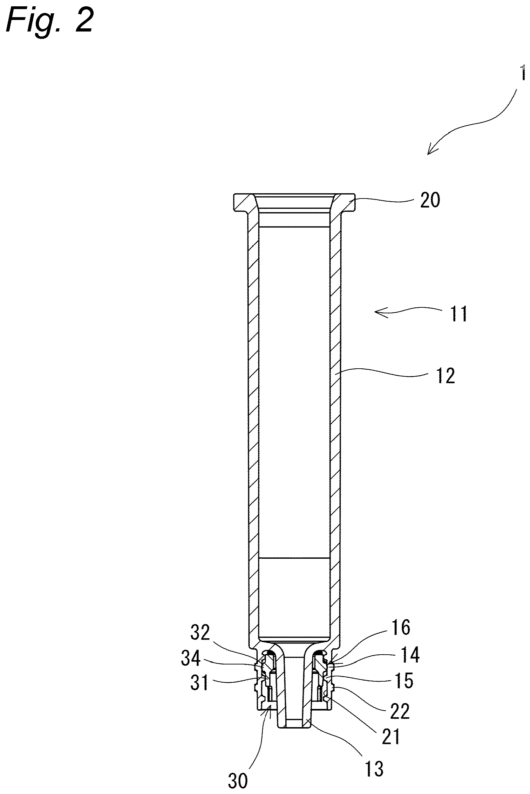

As illustrated in Figs. 1, 2, 10, and 11, a syringe barrel 1 of the present invention includes: a barrel (outer tube portion) 11 having a barrel main body 12 capable of storing a drug, a hollow tip portion 13 provided at a distal portion of the barrel main body 12, and a collar portion 14 that covers the tip portion 13 and includes a barrel-side threaded portion (female screw portion) 15 on an inner surface; and a connection blocking member 30 that is disposed between the tip portion 13 and the collar portion 14 of the barrel 11 and blocks connection of another medical tool (such as an injection needle) to the tip portion 13 and/or the collar portion 14. The connection blocking member 30 includes a cylindrical main body portion 31 and a blocking member-side threaded portion (male screw-shaped portion) 32 that is formed on an outer surface of the cylindrical main body portion 31 and can be screwed with the barrel-side threaded portion 15. In the syringe barrel 1, an inner surface of the collar portion 14 including the barrel-side threaded portion 15 and an outer surface of the cylindrical main body portion 31 including the blocking member-side threaded portion 32 include a rotation restricting engagement portion 16 that is partially engaged when the barrel-side threaded portion 15 and the blocking member-side threaded portion 32 are screwed together and restricts rotation of the connection blocking member 30 in the collar portion 14 in the screwing release direction.

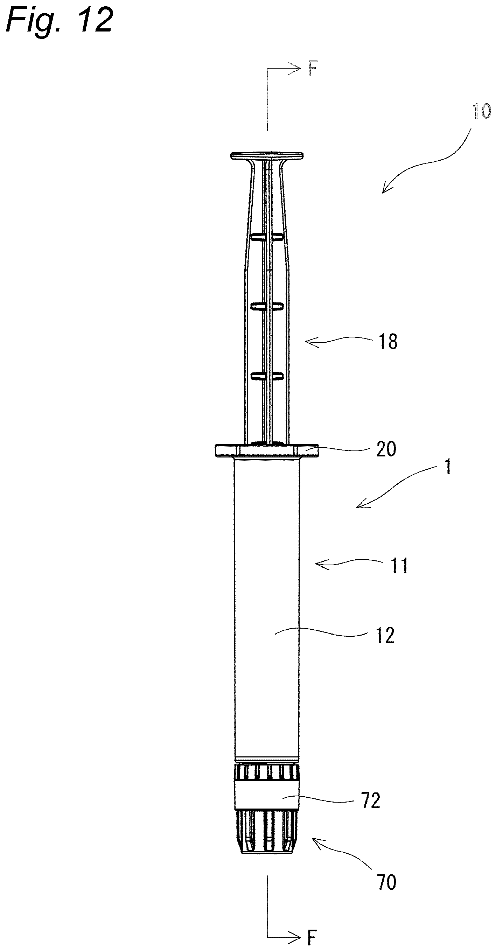

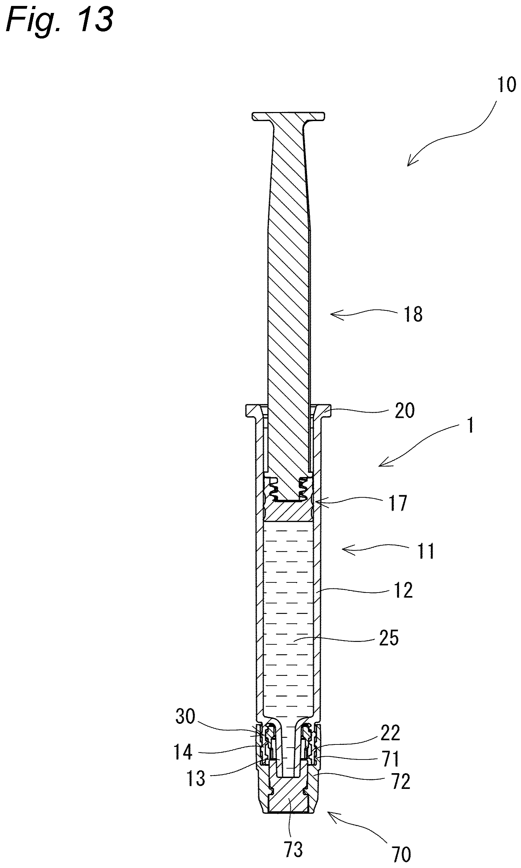

In addition, as illustrated in Figs. 12 and 13, a syringe 10 of the present invention includes the syringe barrel 1 described above, a gasket 17 stored in the syringe barrel 1, and the plunger 18 attached to or attachable to the gasket 17. Note that the syringe of the present invention may be a prefilled syringe in which a drug 25 is stored in advance in a space defined between the inner surface of the syringe barrel 1 (barrel main body 12) and the gasket 17.

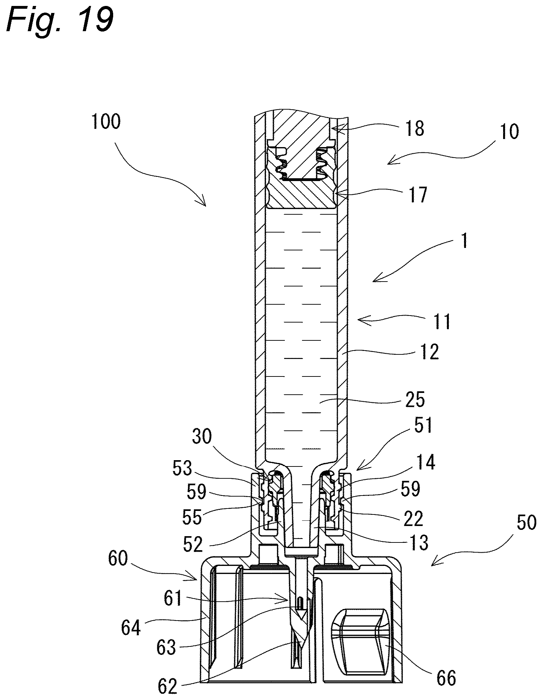

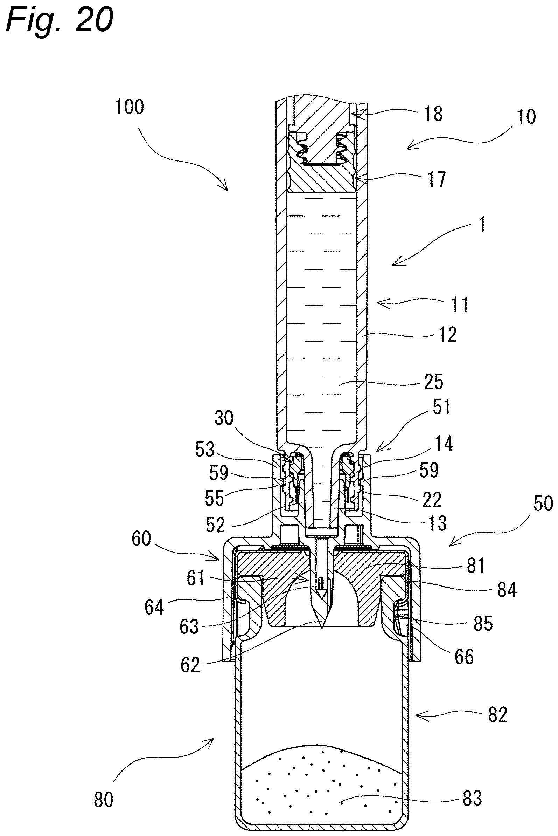

In addition, as illustrated in Figs. 19 and 20, a drug preparation set 100 of the present invention includes the syringe barrel 1 described above and a connection medical tool 50 including a connection portion 51 connectable (detachable) to the tip portion 13 of the syringe barrel 1. The syringe barrel 1 includes a distal end annular space 19 formed between the inner surface of a cylindrical distal portion 33 of the connection blocking member 30 and the outer surface of the tip portion 13 of the barrel 11, and the connection portion 51 of the connection medical tool 50 includes an inner tube portion 52 capable of entering the distal end annular space 19 of the syringe barrel 1 and an outer tube portion 53 covering the inner tube portion 52. The inner tube portion 52 has an inner surface shape that can be liquid-tightly engaged (frictionally engaged) with the outer surface of the tip portion 13, includes an engagement portion 54 that can be engaged with the inner surface of the connection blocking member 30 on the outer surface, and communicates with the inside of the barrel 11 via the tip portion 13 in a connection state between the tip portion 13 of the barrel 11 and the connection medical tool 50. By rotating the syringe barrel 1 or the connection medical tool 50 in a direction in which the screwing of the barrel 11 and the connection blocking member 30 is released in a state in which the engagement portion 54 of the inner tube portion 52 of the connection medical tool 50 is engaged with the inner surface of the connection blocking member 30, the connection blocking member 30 is detached from the barrel 11 together with the connection medical tool 50, and another medical tool (such as an injection needle) can be connected to the tip portion 13 and/or the collar portion 14 (in this example, the tip portion 13 and the collar portion 14 of the barrel 11) of the barrel 11. Note that, in the drug preparation set of the present invention, the syringe barrel may constitute a prefilled syringe in which the drug 25 is stored in advance in a space defined between the inner surface of the syringe barrel 1 (barrel main body 12) and the gasket 17.

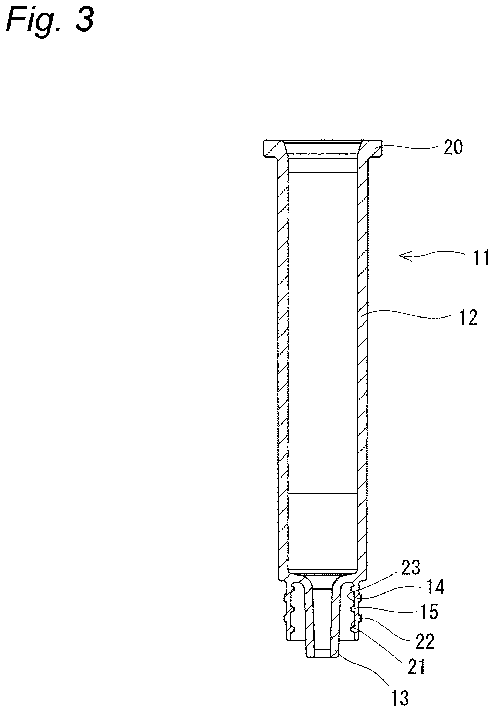

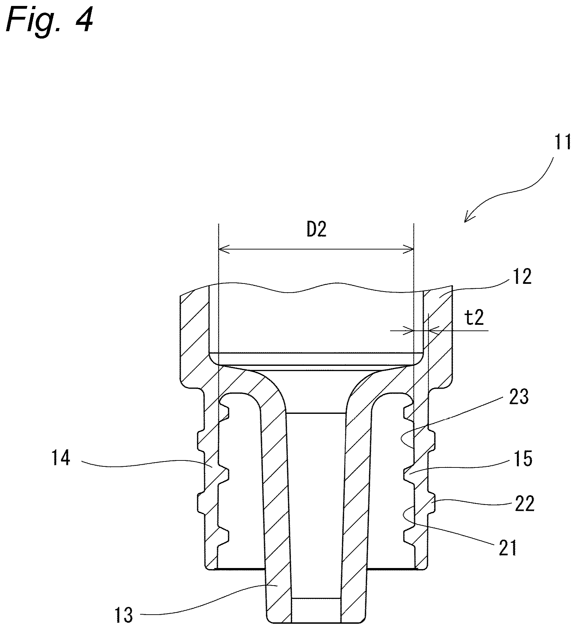

As illustrated in Figs. 3 and 4, the barrel 11 constituting the syringe barrel 1 will be described as a single body (in a state where the connection blocking member 30 is not attached). The barrel 11 includes the barrel main body 12 capable of storing a drug, the hollow tip portion 13 provided at a distal portion of the barrel main body 12, and the collar portion 14 that covers the tip portion 13 and includes the barrel-side threaded portion (female screw portion) 15 on an inner surface.

The barrel main body 12 has a substantially cylindrical shape and has a liquid storage space therein. A flange portion 20 protruding outward is formed at a proximal portion of the barrel main body 12. The tip portion 13 and the collar portion 14 are provided so as to protrude in the distal end direction from the distal portion (shoulder portion) of the barrel main body 12.

The inside of the tip portion 13 communicates with the inside of the barrel main body 12, and the inside of the barrel main body 12 (liquid storage space) communicates with the outside. The outer surface of the tip portion 13 has a tapered shape that narrows in the distal end direction. The tip of the tip portion 13 slightly protrudes in the distal end direction from the collar portion 14.

The collar portion 14 has a substantially cylindrical shape and is formed so as to cover the tip portion 13. A predetermined gap (annular space) is formed between the collar portion 14 (the inner surface of the collar portion 14) and the tip portion 13 (the outer surface of the tip portion 13).

The barrel-side threaded portion 15 is formed on an inner surface of the collar portion 14. In the present example, the barrel-side threaded portion 15 is a spiral recessed portion 21, and can be screwed with a screw rib 34 of the blocking member-side threaded portion 32 described later. The barrel-side threaded portion 15 (spiral recessed portion 21) is formed to continuously extend along the axial direction of the collar portion 14.

A male screw portion 22 is provided on an outer surface of the collar portion 14. The male screw portion 22 is formed to continuously extend along the axial direction of the collar portion 14. The male screw portion 22 can be screwed with a female screw portion 71 of a cap 70 described later and a female screw-shaped portion 55 provided on an inner surface of the outer tube portion 53 of the connection medical tool 50.

The barrel 11 is a cylindrical body formed of a transparent or semitransparent material and, if necessary, a material having low oxygen permeability and water vapor permeability. Examples of the material for forming the barrel 11 include various thermoplastic resins such as polypropylene, polyethylene, polystyrene, polyamide, polycarbonate, polyvinyl chloride, poly- (4-methylpentene-1), acrylic resin, acrylonitrile-butadiene-styrene copolymer, polyester such as polyethylene terephthalate, and cyclic polyolefin. Among these, thermoplastic resins such as polypropylene and cyclic polyolefin are preferable because they are easily molded and have heat resistance.

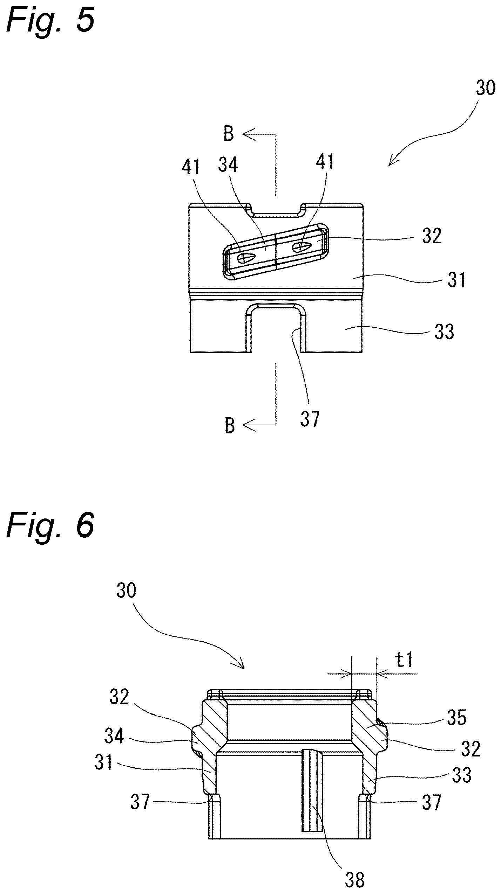

The syringe barrel 1 includes the connection blocking member 30 disposed between the tip portion 13 and the collar portion 14 of the barrel **11.** As illustrated in Figs. 5 to 9, the connection blocking member 30 constituting the syringe barrel 1 will be described as a single body (in a state of not being attached to the barrel 11). The connection blocking member 30 blocks connection of another medical tool (such as an injection needle) to the tip portion 13 and/or the collar portion 14. The connection blocking member 30 may block connection of another medical tool (such as an injection needle) to the collar portion 14, but it is preferable that connection of another medical tool (such as an injection needle) to the tip portion 13 can be blocked. Furthermore, the connection blocking member 30 preferably blocks connection of another medical tool (such as an injection needle) to the tip portion 13 and the collar portion 14.

The connection blocking member 30 is a substantially cylindrical member, and includes the cylindrical main body portion 31 and the blocking member-side threaded portion (male screw-shaped portion) 32 that is formed on an outer surface of the cylindrical main body portion 31 and can be screwed with the barrel-side threaded portion 15. The axial dimension of the connection blocking member 30 is smaller than the axial dimension of the collar portion 14.

As illustrated in Fig. 6, in the cylindrical main body portion 31 of the connection blocking member 30, a portion where the screw rib 34 to be described later is formed is a thick portion 35 having a larger wall thickness than other sites. In other words, in the cylindrical main body portion 31 of the connection blocking member 30, the proximal end side portion (the upper portion in Fig. 6) including the portion where the screw rib 34 is formed is the thick portion 35, and the portion on the distal end side of the thick portion 35 is the cylindrical distal portion 33 having a wall thickness thinner than that of the thick portion 35. A wall thickness t1 of the thick portion 35 is preferably larger than a wall thickness t2 (see Fig. 4) of the collar portion 14. In addition, in the present example, the wall thickness of the distal end side portion of the thick portion 35 gradually decreases toward the cylindrical distal portion 33.

In addition, the cylindrical main body portion 31 of the connection blocking member 30 includes a cutout portion 37 that opens in the distal end direction at the distal end side portion. In the present example, the cylindrical main body portion 31 includes two cutout portions 37 and 37 at portions facing each other in the radial direction. In addition, the cutout portion 37 of the connection blocking member 30 is formed in the cylindrical distal portion 33 of the cylindrical main body portion 31. In other words, the cutout portion 37 of the connection blocking member 30 opens in the distal end direction of the connection blocking member 30 and is formed so as not to reach the thick portion 35. The cutout portion 37 facilitates deformation (for example, spreading) of the distal end side portion of the cylindrical main body portion 31.

In addition, an inner surface of the connection blocking member 30 is provided with a blocking member-side longitudinal rib 38 engageable with the engagement portion 54 (medical tool-side longitudinal rib 56) formed on an outer surface of the inner tube portion 52 of the connection medical tool 50 described later. Specifically, as illustrated in Figs. 6 to 8, on the inner surface of the connection blocking member 30 (cylindrical main body portion 31), two blocking member-side longitudinal ribs 38 and 38 are formed at portions facing each other in the radial direction. The blocking member-side longitudinal rib 38 protrudes inward from the inner surface of the cylindrical main body portion 31 and extends in the axial direction of the cylindrical main body portion 31. In addition, the blocking member-side longitudinal rib 38 of the connection blocking member 30 extends from a portion slightly on the proximal end side with respect to the distal end of the cylindrical main body portion 31 toward the proximal end direction and is formed to abut on the thick portion 35 (the portion where the wall thickness gradually decreases toward the cylindrical distal portion 33 described above).

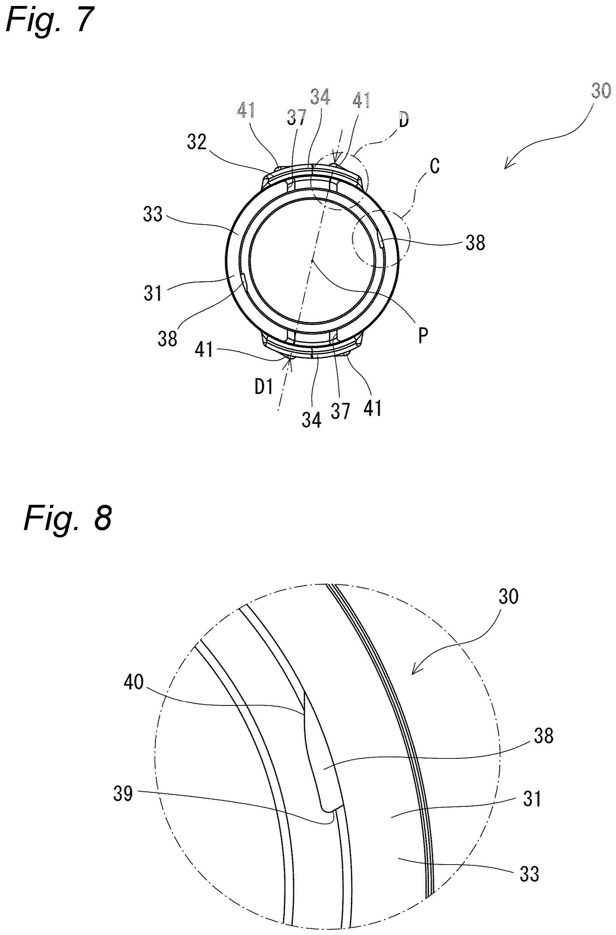

In the present example, as illustrated in Figs. 7 and 8, the side surface on one side in the circumferential direction of the blocking member-side longitudinal rib 38 (the side surface on the clockwise direction side in Fig. 7) is an engagement surface 39 rising inward from the inner surface of the connection blocking member 30 (cylindrical main body portion 31), and the side surface on the other side in the circumferential direction (the side surface on the half clockwise direction side in Fig. 7) is an inclined surface 40 rising inwardly from the inner surface of the connection blocking member 30 (cylindrical main body portion 31). As a result, as will be described later, the blocking member-side longitudinal rib 38 constitutes an uneven structure that can be engaged with each other on the inner surface of the connection blocking member 30 and the outer surface of the inner tube portion 52 of the connection medical tool 50 together with the engagement portion 54 (medical tool-side longitudinal rib 56) provided on the connection medical tool 50, and constitutes a ratchet structure that rotates the connection blocking member 30 only in the direction of detachment (screwing) from the barrel 11.

The blocking member-side threaded portion (male screw-shaped portion) 32 is formed on an outer surface of the cylindrical main body portion 31. The blocking member-side threaded portion 32 of the connection blocking member 30 is the screw rib 34 that can be screwed with the spiral recessed portion 21 of the barrel 11. In the present example, the screw rib 34 of the connection blocking member 30 is a short spiral rib extending along the axial direction of the connection blocking member 30. Specifically, as illustrated in Figs. 6 and 7, two screw ribs 34 and 34 are provided at portions facing each other in the radial direction of the connection blocking member 30, and each has a length of about 1/6 circumference in the circumferential direction.

The connection blocking member 30 includes a protruding portion 41 provided on the upper surface of the screw rib 34 forming the blocking member-side threaded portion 32. As illustrated in Fig. 7, the protruding portion 41 protrudes outward from the upper surface of the screw rib 34. A diameter (in the present example, the distance between the vertices of the two protruding portions 41 facing each other in the radial direction) D1 of a portion of the connection blocking member 30 where the protruding portion 41 is formed is larger than an inner diameter D2 (see Fig. 4) of the barrel-side threaded portion 15 (spiral recessed portion 21).

In the present example, the two screw ribs 34 and 34 are provided with two protruding portions 41 and 41, respectively. Further, as illustrated in Fig. 7, it is preferable that the protruding portions 41 provided on the two screw ribs 34 and 34 are provided at portions facing each other in the radial direction, and it is more preferable that a straight line connecting the vertices of the two protruding portions 41 and 41 facing each other passes through the center P of the connection blocking member 30. Here, in each of the two screw ribs 34 and 34, the protruding portions 41 and 41 provided on one circumferential direction side (clockwise direction side in Fig. 7) and the protruding portions 41 and 41 provided on the other circumferential direction side (counterclockwise direction side in Fig. 7) are provided at portions facing each other in the radial direction, and a straight line connecting vertices of the two protruding portions 41 and 41 facing each other passes through the center P of the connection blocking member 30.

It is preferable that the protruding portion 41 has a small protrusion amount and/or a small width in the screwing direction (starting end direction) of the screw rib 34 of the connection blocking member 30. In the present example, as illustrated in Figs. 5, 7, and 9, the protruding portion 41 has a small protrusion amount and a small width in the screwing direction (starting end direction or clockwise direction in Fig. 7) of the screw rib 34, and has a substantially drop-like shape (shape like a droplet of water) in a case where the protruding portion 41 is viewed from above (see Fig. 5).

Examples of the material for forming the connection blocking member 30 include various thermoplastic resins. Then, the connection blocking member 30 is preferably formed of a thermoplastic resin harder than the thermoplastic resin forming the barrel 11. In addition, the connection blocking member 30 is preferably formed of a thermoplastic resin having a higher thermal deformation temperature (softening point or glass transition point) than the thermoplastic resin forming the barrel 11. For example, in a case where the barrel 11 is made of polypropylene, the connection blocking member 30 can be made of polycarbonate. In addition, in a case where the barrel 11 is made of cyclic polyolefin, the connection blocking member 30 can be made of glass fiber-reinforced polycarbonate.

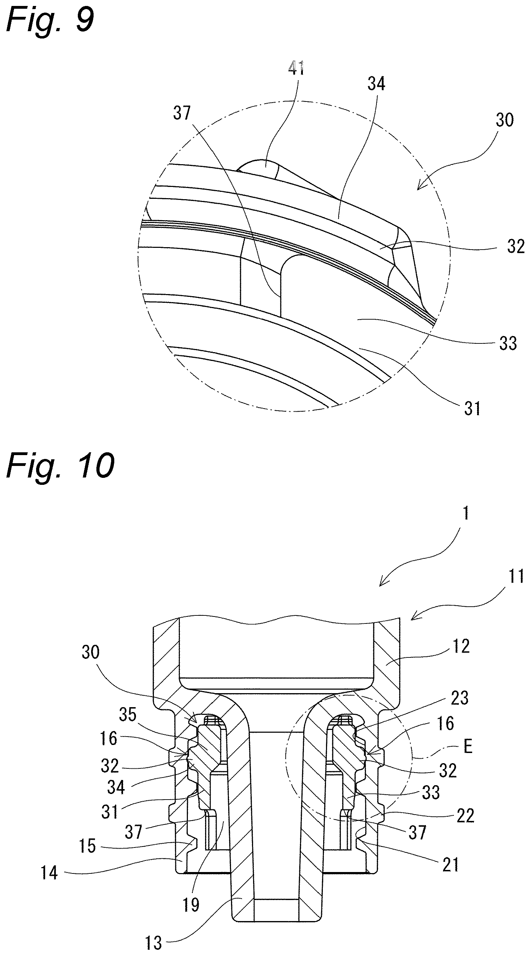

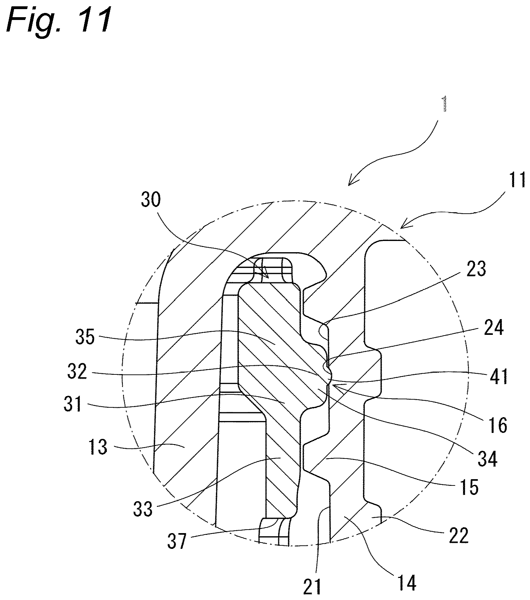

Next, the syringe barrel 1 in a state where the connection blocking member 30 is attached to the barrel 11 will be described as illustrated in Figs. 10 and 11.

The inner surface of the collar portion 14 including the barrel-side threaded portion 15 and the outer surface of the cylindrical main body portion 31 including the blocking member-side threaded portion 32 of the syringe barrel 1 are partially engaged with each other when the barrel-side threaded portion 15 and the blocking member-side threaded portion 32 are screwed (when the connection blocking member 30 is attached to the barrel 11), and include the rotation restricting engagement portion 16 that restricts the rotation of the connection blocking member 30 in the collar portion 14 in the screwing release direction.

Specifically, in the present example, as illustrated in Figs. 10 and 11, the connection blocking member 30 includes the protruding portion 41 provided on the upper surface of the screw rib 34 forming the blocking member-side threaded portion 32, the spiral recessed portion 21 forming the barrel-side threaded portion 15 includes the inner surface site 23 engaged with the protruding portion 41, and the rotation restricting engagement portion 16 is formed by the protruding portion 41 and the inner surface site 23 of the spiral recessed portion 21.

More specifically, the inner surface site 23 of the spiral recessed portion 21 (barrel-side threaded portion 15) includes a recessed portion 24 which is engaged with the protruding portion 41 of the connection blocking member 30 and stores a vertex portion of the protruding portion 41. In other words, in the present example, the protruding portion 41 of the connection blocking member 30 and the recessed portion 24 of the barrel 11 are engaged with each other to form the rotation restricting engagement portion 16 that restricts the rotation of the connection blocking member 30 in the screwing release direction in the collar portion 14. Note that the recessed portion 24 may be formed in the barrel 11 in advance, formed by attaching the connection blocking member 30 to the barrel 11, or formed by heating (for example, high-pressure steam sterilization) after attaching the connection blocking member 30 to the barrel **11.**

In addition, as illustrated in Fig. 10, the syringe barrel 1 includes the distal end annular space 19 formed between the inner surface of the cylindrical distal portion 33 of the connection blocking member 30 and the outer surface of the tip portion 13 of the barrel 11. In the present example, in the connection blocking member 30, the inner diameter of the cylindrical distal portion 33 is larger than the inner diameter of the thick portion 35 (in other words, the thick portion 35 has a wall thickness so as to reduce the inner diameter), and in such a cylindrical distal portion 33, the distal end annular space 19 is formed between the inner surface of the connection blocking member 30 and the outer surface of the tip portion 13.

Note that, as illustrated in Figs. 12 and 13, the syringe barrel 1 may include the cap 70 that seals the tip portion 13, the barrel 11 may include the male screw portion 22 provided on an outer surface of the collar portion 14, and the cap 70 may include a cap main body portion 72 having the female screw portion 71 screwed to the male screw portion 22 of the collar portion 14, and a sealing member 73 that is supported by the cap main body portion 72 and abuts on a distal portion of the tip portion 13 to seal the tip portion 13.

A method for manufacturing such a syringe barrel 1 will be described. A method for manufacturing the syringe barrel 1 described below includes: a step of preparing the barrel 11 and the connection blocking member 30; a connection blocking member mounting step of disposing the connection blocking member 30 between the tip portion 13 and the collar portion 14 by screwing, and engaging the protruding portion 41 provided on the upper surface of the screw rib 34 forming the blocking member-side threaded portion 32 with a part (inner surface site 23) of the spiral recessed portion 21 forming the barrel-side threaded portion 15; and a heating step of heating the barrel 11 on which the connection blocking member 30 is mounted by the connection blocking member mounting step, and embedding the vertex site of the protruding portion 41 of the connection blocking member 30 in the inner surface of the spiral recessed portion 21 forming the barrel-side threaded portion 15 by deformation of the barrel 11 due to heating.

In the present example, the above-described recessed portion 24 is not yet formed in the barrel 11 prepared in the step of preparing the barrel 11 and the connection blocking member 30. Then, as the connection blocking member 30, a member formed of a thermoplastic resin that is harder than the barrel 11 and has a higher thermal deformation temperature (softening point or glass transition point) is prepared.

In addition, the connection blocking member mounting step can be performed by attaching a tool for rotating (screwing) the connection blocking member 30 to the cutout portion 37 of the connection blocking member 30. As a result, it is possible to more easily screw the connection blocking member 30 into the barrel 11 and engage the protruding portion 41 with the spiral recessed portion 21 (inner surface site 23). In addition, in the present example, since the protruding portion 41 has a small protrusion amount and a small width in the screwing direction of the screw rib 34 of the connection blocking member 30, the screw rib 34 including the protruding portion 41 easily enters (is screwed into) the spiral recessed portion 21.

In the present example, the diameter (outer diameter) D1 of the portion of the connection blocking member 30 where the protruding portion 41 is formed is larger than the inner diameter D2 of the barrel-side threaded portion 15 (spiral recessed portion 21). In addition, the wall thickness t1 of the thick portion 35 where the screw rib 34 of the connection blocking member 30 is formed is thicker than the wall thickness t2 of the collar portion 14. Therefore, the protruding portion 41 provided on the upper surface of the screw rib 34 engages with a part of the spiral recessed portion 21 in a state where the collar portion 14 is elastically deformed. Note that, in the connection blocking member mounting step, the engagement between the protruding portion 41 and the spiral recessed portion 21 may be performed only by the elastic deformation of the collar portion 14, or the spiral recessed portion 21 (inner surface site 23) may be slightly deformed (recessed).

Next, a heating step is performed in which the barrel 11 to which the connection blocking member 30 is mounted is heated by the connection blocking member mounting step, and the vertex site of the protruding portion 41 of the connection blocking member 30 is embedded in the inner surface (inner surface site 23) of the spiral recessed portion 21 forming the barrel-side threaded portion 15 by the deformation of the barrel 11. Note that, in the present example, the heating step is performed by high-pressure steam sterilization.

Specifically, in the present example, the vertex site of the protruding portion 41 and the inner surface (inner surface site 23) of the spiral recessed portion 21 are pressed against each other in a state where an elastic force due to elastic deformation of the collar portion 14 acts by the connection blocking member mounting step. Therefore, by heating the barrel 11 to which the connection blocking member 30 is attached, as illustrated in Figs. 10 and 11, the inner surface (inner surface site 23) of the spiral recessed portion 21 is deformed (thermally deformed), and the recessed portion 24 for storing the vertex portion of the protruding portion 41 is formed in the inner surface site 23. In addition, in the present example, the connection blocking member 30 is made of a thermoplastic resin that is harder and has a higher thermal deformation temperature (softening point or glass transition point) than the barrel 11. As a result, in the heating step, the barrel 11 (the inner surface site 23 of the spiral recessed portion 21) is deformed (thermally deformed) more preferentially than the connection blocking member 30 (the protruding portion 41), and the recessed portion 24 can be more reliably formed in the barrel 11, in other words, the protruding portion 41 of the connection blocking member 30 can be bitten into the barrel 11 (the inner surface site 23 of the spiral recessed portion 21).

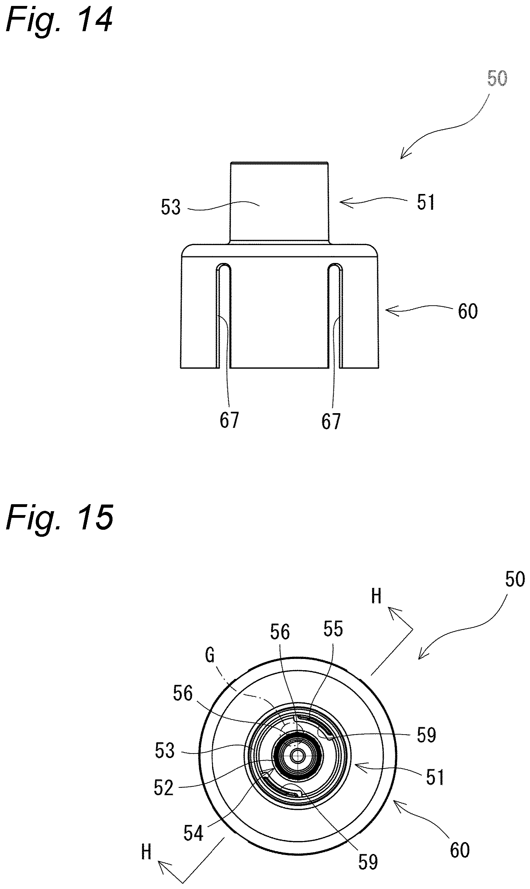

The connection medical tool 50 constituting the drug preparation set 100 together with the syringe barrel 1 described above will be described as illustrated in Figs. 14 to 17.

In the present example, the connection medical tool 50 is a vial adapter. Specifically, as illustrated in Fig. 20, the connection medical tool 50 is a vial adapter for connecting two medical tools, here, the syringe barrel 1 (prefilled syringe) and a vial 80, and enabling distribution of a drug or the like therebetween. Note that the connection medical tool is not limited to a vial adapter, and may be a port adapter connectable to a port provided in a medicinal solution bag, or a syringe including the connection portion 51 connectable (detachable) to a tip portion of a syringe barrel as described later.

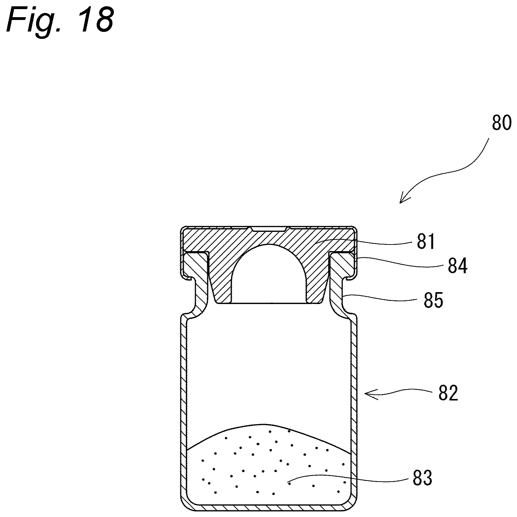

Note that the vial 80 of the present example is a general vial, and as illustrated in Fig. 18, a drug 83 is stored in a glass container 82 sealed with a rubber stopper 81. The peripheral edge portion of the rubber stopper 81 and the peripheral edge portion of the opening of the container 82 are covered with a covering member 84. The covering member 84 is preferably formed of aluminum, a heat-shrinkable film, or the like, and is in close contact with the rubber stopper 81 and the container 82. Note that examples of the drug stored in the vial include a powder (for example, a lyophilized preparation or a powder preparation) and a liquid. Then, at the time of administration of the drug, the drug for administration (medicinal solution) can be prepared by injecting a medical liquid such as a solution or a diluent into the vial.

As illustrated in Figs. 14 to 17, the connection medical tool 50 includes the connection portion 51 connectable (detachable) to the tip portion 13 of the syringe barrel 1 described above. The connection portion 51 includes the inner tube portion 52 capable of entering the distal end annular space of the syringe barrel 1 and the outer tube portion 53 that covers the inner tube portion 52. The connection portion 51 constitutes a substantially upper half portion (upper portion in Fig. 14) of the connection medical tool 50.

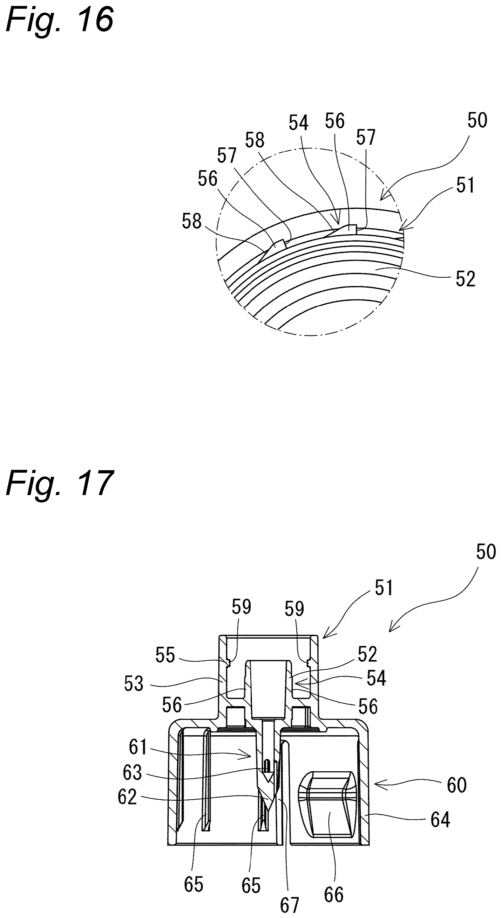

The inner tube portion 52 of the connection portion 51 has an inner surface shape that can be liquid-tightly engaged with the outer surface of the tip portion 13. Specifically, as illustrated in Fig. 17, the inner surface shape of the inner tube portion 52 is a tapered recessed shape corresponding to the outer surface shape of the tapered tip portion 13. In addition, the inner tube portion 52 communicates with the inside of the barrel 11 (liquid storage space) in a connection state with the tip portion 13 of the barrel 11.

The engagement portion 54 engageable with an inner surface of the connection blocking member 30 is formed on an outer surface of the inner tube portion 52. Specifically, a plurality of (here, 16) medical tool-side longitudinal ribs 56 engageable with the blocking member-side longitudinal ribs 38 formed on the inner surface of the connection blocking member 30 described above is formed on the outer surface of the inner tube portion 52. The plurality of medical tool-side longitudinal ribs 56 is preferably formed at equal intervals in the circumferential direction. The medical tool-side longitudinal rib 56 protrudes outward from the outer surface of the inner tube portion 52 and extends in the axial direction of the inner tube portion 52.

In the present example, as illustrated in Figs. 15 and 16, a side surface on one side in the circumferential direction (a side surface on the clockwise direction side in Fig. 15) of the medical tool-side longitudinal rib 56 is an engagement surface 57 rising outward from the outer surface of the inner tube portion 52, and a side surface on the other side in the circumferential direction (a side surface on the half clockwise direction side in Fig. 15) is an inclined surface 58 rising inclinedly outward from the outer surface of the inner tube portion 52. As a result, the medical tool-side longitudinal rib 56 and the above-described blocking member-side longitudinal rib 38 constitute a ratchet structure that rotates the connection blocking member 30 only in the direction of detachment (screwing) from the barrel 11.

The connection portion 51 includes the outer tube portion 53 that covers the inner tube portion 52. An inner diameter of the outer tube portion 53 is larger than an outer diameter of the collar portion 14 of the barrel 11, and the collar portion 14 can be stored between the inner tube portion 52 (an outer surface of the inner tube portion 52) and the outer tube portion 53 (an inner surface of the outer tube portion 53).

On the inner surface of the outer tube portion 53, the female screw-shaped portion 55 that can be screwed into the male screw portion 22 provided on the outer surface of the collar portion 14 described above is formed. In the present example, the female screw-shaped portion 55 is a part of a spiral rib (female screw) extending along the axial direction of the outer tube portion 53, and includes two female screw portion constituting ribs 59 and 59 provided at portions facing each other in the radial direction of the outer tube portion 53, and these female screw portion constituting ribs 59 are each formed to have a length of about 1/6 circumference in the circumferential direction.

The connection medical tool 50 includes a vial mounting portion 60 for mounting (connecting) the connection medical tool 50 to the vial 80 in a substantially lower half portion (lower portion in Fig. 14) different from the portion constituting the connection portion 51.

As illustrated in Fig. 17, the vial mounting portion 60 includes a hollow needle-shaped portion 61 that can penetrate the rubber stopper 81 of the vial 80 and can communicate the inside of the vial 80 with the inner tube portion 52. The hollow needle-shaped portion 61 extends downward (downward in Fig. 17) from the bottom portion of the inner tube portion 52 (tapered recessed portion). The hollow needle-shaped portion 61 includes a sharp distal portion 62 that can enter the rubber stopper 81 of the vial 80 and pass through the rubber stopper 81, and an opening 63 that communicates the inside and the outside of the hollow needle-shaped portion 61. Note that, in the present example, the opening 63 is opened in the side surface of the hollow needle-shaped portion 61, and foreign matter (fragments of the rubber stopper 81 or the like) hardly enters when the hollow needle-shaped portion 61 passes through the rubber stopper 81.

The vial mounting portion 60 includes a cylindrical vial covering portion 64. On the inner surface of the vial covering portion 64, a plurality of (here, six) longitudinal ribs 65 and a plurality of (here, two) retaining protruding portions 66 are provided. In addition, a plurality of (here, four) slits 67 is formed in the vial covering portion 64, and a portion of the vial covering portion 64 between the slits 67 is elastically deformable in the radial direction. In the present example, the vial mounting portion 60 is mounted on the vial 80 in a state where the vial covering portion 64 is elastically deformed (using the elastic force of the vial covering portion 64). In addition, the retaining protruding portion 66 can be engaged with an annular recessed portion 85 formed in the upper portion (upper portion in Fig. 18) of the vial 80, and the connection medical tool 50 can be prevented from being unintentionally detached from the vial 80.

Note that, in the present example, the connection medical tool 50 including the connection portion 51 and the vial mounting portion 60 is integrally formed of a resin material.

A general method of preparing a drug using the drug preparation set 100 including the syringe barrel 1 and the connection medical tool 50 as described above will be briefly described with reference to Figs. 19 to 23 and the following.

First, the syringe barrel 1 is provided, and the syringe (prefilled syringe) 10 in which the drug (first drug that is a liquid drug) 25 is stored in advance is prepared.

Next, as illustrated in Fig. 19, the syringe 10 is connected to the connection medical tool 50 (connection portion 51). Specifically, the female screw-shaped portion 55 of the outer tube portion 53 of the connection medical tool 50 and the male screw portion 22 of the collar portion 14 of the syringe 10 are threadedly engaged with each other, and the inner surface of the inner tube portion 52 of the connection medical tool 50 and the outer surface of the tip portion 13 of the syringe 10 are liquid-tightly engaged (frictionally engaged) with each other. At this time, the engagement portion 54 (medical tool-side longitudinal rib 56) of the inner tube portion 52 of the connection medical tool 50 and the blocking member-side longitudinal rib 38 of the connection blocking member 30 of the syringe 10 come into contact with each other, but due to the ratchet structure (sliding between the inclined surfaces 40 and 58), the connection blocking member 30 is prevented from rotating along with the rotation of the connection medical tool 50 with respect to the syringe 10.

Next, as illustrated in Fig. 20, the connection medical tool 50 to which the syringe 10 is connected is attached to the vial 80. As a result, the inside of the vial 80 communicates with the outside via the connection medical tool 50 (the hollow needle-shaped portion 61 and the inner tube portion 52). Note that, in the vial 80, the drug 83 (second drug that is a powder) is stored. In addition, the vial 80 of the present example is a so-called decompressed vial whose inside is decompressed.

By attaching the connection medical tool 50 to which the syringe 10 is connected to the vial 80, the syringe 10 and the vial 80 communicate with each other via the connection medical tool 50 (the hollow needle-shaped portion 61 and the inner tube portion 52). Then, the plunger 18 of the syringe 10 is operated to inject the first drug in the syringe 10 into the vial 80, and the first drug and the second drug are mixed in the vial 80, thereby preparing a drug for administration. Then, the prepared drug for administration is stored in the syringe 10 again.

Next, the syringe 10 is detached from the connection medical tool 50. At this time, the connection blocking member 30 is detached from the barrel 11 together with the connection medical tool 50.

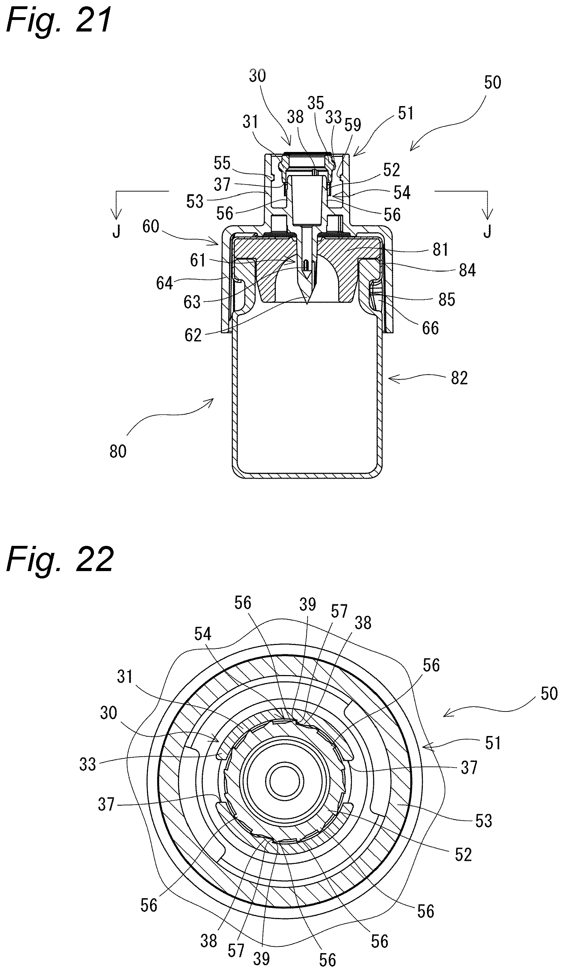

Specifically, as illustrated in Figs. 21 and 22, in the present example, in order to detach the syringe 10 from the connection medical tool 50, by rotating the syringe barrel 1 or the connection medical tool 30 in a direction in which the screwing between the syringe 10 (barrel 11) and the connection blocking member 30 is released, the engagement portion 54 (medical tool-side longitudinal rib 56) of the inner tube portion 52 of the connection medical tool 50 and the blocking member-side longitudinal rib 38 of the connection blocking member 30 of the syringe 10 are engaged with each other on the engagement surfaces 39 and 57 (see Fig. 22), and the connection blocking member 30 is rotated in a direction of detaching (screwing) from the barrel 11. As a result, the connection blocking member 30 can be detached from the barrel 11 as the connection medical tool 50 is detached from the barrel 11.

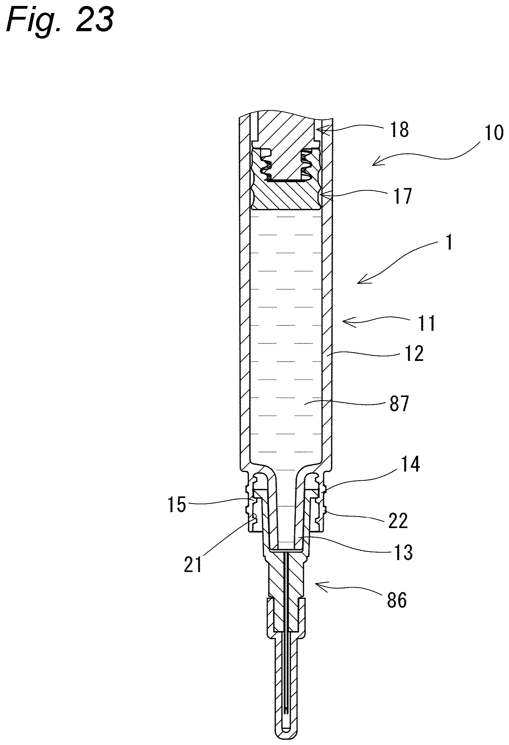

Next, as illustrated in Fig. 23, by connecting the injection needle 86 for administration as another medical tool to the tip portion 13 and/or the collar portion 14 (in this example, the tip portion 13 and the collar portion 14 of the barrel 11) of the syringe 10 from which the connection blocking member 30 has been detached, the prepared drug 87 can be administered to a patient.

The syringe barrel 1, the syringe 10, and the drug preparation set 100 of the present example include the connection blocking member 30 that blocks connection of another medical tool (such as an injection needle) to the tip portion 13 and/or the collar portion 14. In the syringe barrel 1, the inner surface of the collar portion 14 including the barrel-side threaded portion 15 and the outer surface of the cylindrical main body portion 31 including the blocking member-side threaded portion 32 partially engage with each other at the time of screwing the barrel-side threaded portion 15 and the blocking member-side threaded portion 32, and include the rotation restricting engagement portion 16 that restricts rotation of the connection blocking member 30 in the collar portion 14 in the screwing release direction. As a result, the connection blocking member 30 is less likely to fall off, and erroneous connection (erroneous administration) can be more reliably prevented by the connection blocking member 30.

In addition, according to the method for manufacturing a syringe barrel of the present example, the syringe barrel 1 as described above can be more easily manufactured.

Note that, in the above-described example, the recessed portion 24 is formed by the heating step, but the recessed portion 24 may be formed by attaching the connection blocking member 30 to the barrel **11.** Further, the recessed portion 24 may be formed in advance (before attachment of the connection blocking member 30). In addition, a preliminary engagement recessed portion shallower than the finally formed recessed portion 24 may be formed in a portion where the recessed portion 24 is to be formed or a portion where the recessed portion 24 is desired to be formed with respect to the barrel **11.**

Note that, in the above example, the protrusion amount and the width of the protruding portion 41 decrease in the screwing direction (starting end direction) of the screw rib 34, but the present invention is not limited to such a form. In addition, the protruding portion 41 has a substantially drop-like shape (shape like a droplet of water) when viewed from above, but is not limited to such a shape, and may have a hemispherical shape, a fan shape, or a linear rib shape, for example.

Note that the other medical tool connected to the tip portion 13 and/or the collar portion 14 after detachment of the connection blocking member 30 is not limited to the injection needle 86 described above, and may be, for example, a three-way stopcock connected to an infusion tube.

Note that, in the above example, the heating step is performed by high-pressure steam sterilization, and heating and sterilization are simultaneously performed, but the present invention is not limited thereto. For example, after simply performing the heating step on the barrel 11 to which the connection blocking member 30 is attached, another sterilization method (sterilization with, for example, gamma radiation, EOG, and hydrogen peroxide) can be performed. In addition, as described above, in a case where the recessed portion 24 is formed in advance, the heating step may not be performed.

Hereinafter, another embodiment of a syringe barrel of the present invention will be described with reference to the drawings. Note that, in the following description, configurations substantially similar to those of the syringe barrel 1 described above will be referred to by using corresponding names and reference signs.

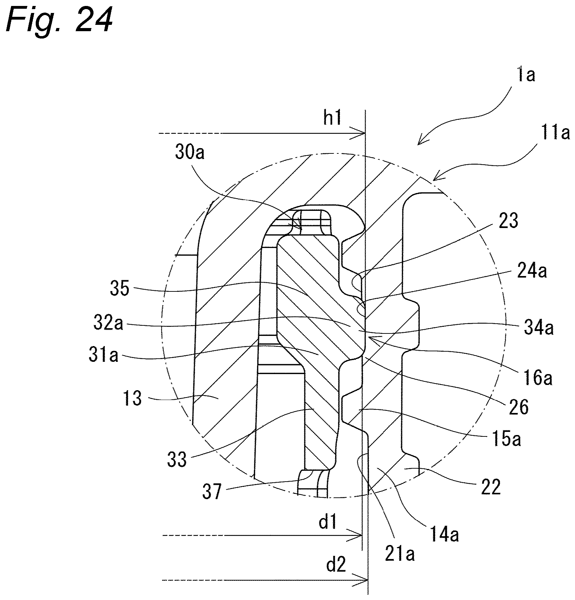

In the syringe barrel 1a illustrated in Fig. 24, the connection blocking member 30a includes a screw rib 34a forming the blocking member-side threaded portion 32a, and a spiral recessed portion 21a forming a barrel-side threaded portion 15a includes a low recessed site 26 having a recessed portion depth shallower than other sites. Then, the low recessed site 26 and the outer surface of the screw rib 34a form a rotation restricting engagement portion 16a.

Specifically, as illustrated in Fig. 24, the low recessed site 26 is provided in a proximal end side portion of the spiral recessed portion 21a forming the barrel-side threaded portion 15a of the barrel 11a. The recessed portion depth d1 of the low recessed site 26 (distance from the center of the collar portion 14a) is shallower (smaller) than the recessed portion depth d2 of the other site of the spiral recessed portion 21a. In addition, in the present example, the protruding portion as described above is not provided in the screw rib 34a of the connection blocking member 30a, and the protruding portion height h1 of the screw rib 34a (distance from the center of the cylindrical main body portion 31a) is larger (higher) than the recessed portion depth d1 of the low recessed site 26. As a result, the inner surface (low recessed site 26) of the collar portion 14a and the outer surface (upper surface of the screw rib 34a) of the cylindrical main body portion 31a are strongly engaged (abutted), and a rotation restricting engagement portion 16a that restricts the rotation of the connection blocking member 30a in the collar portion 14a in the screwing release direction is formed. Note that, in the present example, the rotation restricting engagement portion 16a (low recessed site 26) has the recessed portion 24a for storing the upper portion of the screw rib 34a, but such a recessed portion may not be formed. In addition, even in a case where the syringe barrel 1a includes such a low recessed site 26, a protruding portion may be provided in the screw rib 34a of the connection blocking member 30a.

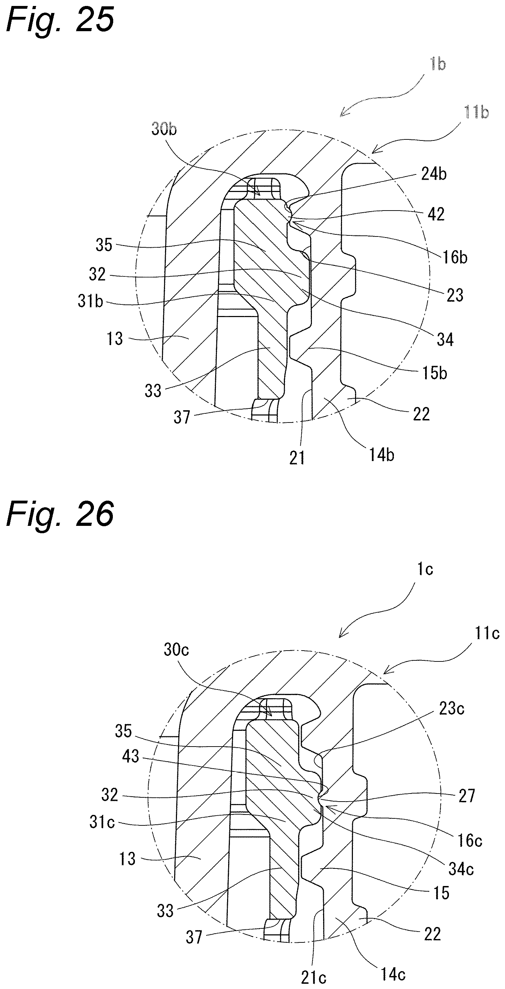

In the syringe barrel 1b illustrated in Fig. 25, a protruding portion 42 is formed on an outer surface of the connection blocking member 30b at a portion where the screw rib 34 is not formed. As a result, the inner surface (the upper surface of the crest portion of the barrel-side threaded portion 15b, in other words, the inner surface of the collar portion 14b, where the spiral recessed portion 21 is not formed) of the collar portion 14b and the outer surface (protruding portion 42) of the cylindrical main body portion 31b are strongly engaged (abutted), and a rotation restricting engagement portion 16b that restricts the rotation of the connection blocking member 30b in the screwing release direction in the collar portion 14b is formed. Note that, in the present example, the rotation restricting engagement portion 16b (the upper surface of the crest portion of the barrel-side threaded portion 15b of the barrel 11b) is formed with the recessed portion 24b that stores the vertex portion of the protruding portion 42, but such a recessed portion may not be formed. Note that, in order to more reliably form the rotation restricting engagement portion 16b, a plurality of the protruding portions 42 may be formed in the circumferential direction of the connection blocking member 30b or may be formed over the entire circumference.

In a syringe barrel 1c illustrated in Fig. 26, a protruding portion 27 is formed in an inner surface site 23c of a spiral recessed portion 21c in a collar portion 14c of a barrel 11c. As a result, the inner surface (protruding portion 27) of the collar portion 14c and the outer surface (the upper surface of the screw rib 34c) of the cylindrical main body portion 31c are strongly engaged (abutted), and a rotation restricting engagement portion 16c that restricts the rotation of the connection blocking member 30c in the collar portion 14c in the screwing release direction is formed. Note that, in the present example, the rotation restricting engagement portion 16c (the upper surface of the screw rib 34c) has the recessed portion 43 for storing the vertex portion of the protruding portion 27, but such a recessed portion may not be formed. Note that, in order to more reliably form the rotation restricting engagement portion 16c, a plurality of the protruding portions 27 may be formed in the circumferential direction of the collar portion 14c or may be formed over the entire circumference. Note that, in the case of this example, the barrel 11c is preferably formed of a thermoplastic resin harder than the thermoplastic resin forming the connection blocking member 30c.

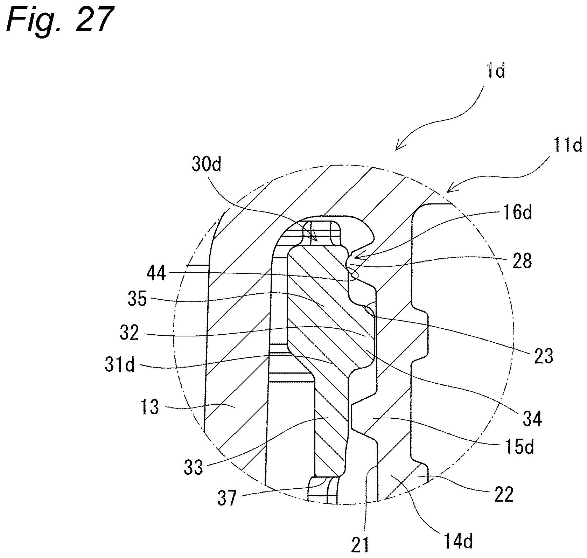

In a syringe barrel 1d illustrated in Fig. 27, the protruding portion 28 is formed on the upper surface of the crest portion of a barrel-side threaded portion 15d of a collar portion 14d of a barrel 11d (a portion where the spiral recessed portion 21 is not formed on the inner surface of the collar portion 14d). As a result, the inner surface (protruding portion 28) of the collar portion 14d and the outer surface of a cylindrical main body portion 31d (the outer surface of a connection blocking member 30d and the portion where the screw rib 34 is not formed) are strongly engaged (abutted) to form a rotation restricting engagement portion 16d that restricts the rotation of the connection blocking member 30d in the screwing release direction in the collar portion 14d. Note that, in the present example, in the rotation restricting engagement portion 16d (the outer surface of the connection blocking member 30d and the portion where the screw rib 34 is not formed), the recessed portion 44 for storing the vertex portion of the protruding portion 28 is formed, but such a recessed portion may not be formed. Note that, in the case of this example, the barrel 11d is preferably formed of a thermoplastic resin harder than the thermoplastic resin forming the connection blocking member 30d.

Further, in the syringe barrel, the screw rib forming the blocking member-side threaded portion may include a raised portion formed higher than other portions, the spiral recessed portion forming the barrel-side threaded portion may include an inner surface site to be engaged with the raised portion, and the rotation restricting engagement portion may be formed by the raised portion and the inner surface site of the spiral recessed portion. Note that it is preferable that the protrusion amount and/or the width of such a raised portion decrease in the screwing direction (starting end direction) of the screw rib of the connection blocking member.

Note that, in the above-described example, the uneven structure that can be engaged with each other is formed by the inner surface (blocking member-side longitudinal rib formed on inner surface and extending in axial direction) of the connection blocking member and the outer surface (a medical tool-side longitudinal rib formed on the outer surface and extending in the axial direction) of the inner tube portion of the connection medical tool, but the present invention is not limited thereto. For example, such an uneven structure may include an annular protrusion extending in the circumferential direction.

A syringe barrel, a syringe, and a drug preparation set of the present invention include a connection blocking member that blocks connection of another medical tool (such as an injection needle) to a tip portion and/or a collar portion, and in the syringe barrel, an inner surface of the collar portion including a barrel-side threaded portion and an outer surface of a cylindrical main body portion including a blocking member-side threaded portion partially engage with each other at the time of screwing of the barrel-side threaded portion and the blocking member-side threaded portion, and the syringe barrel includes a rotation restricting engagement portion that restricts rotation of the connection blocking member in the collar portion in a screwing release direction. As a result, the connection blocking member is more unlikely to fall off, and erroneous connection (erroneous administration) can be more reliably prevented by the connection blocking member.

In addition, according to the method for manufacturing a syringe barrel of the present example, the syringe barrel as described above can be more easily manufactured.

## Claims

1. A syringe barrel comprising: a barrel having a barrel main body capable of storing a drug, a hollow tip portion provided at a distal portion of the barrel main body and a collar portion covering the tip portion and comprising a barrel-side threaded portion on an inner surface; and a connection blocking member disposed between the tip portion and the collar portion of the barrel and blocking connection of another medical tool to the tip portion and/or the collar portion, wherein
the connection blocking member comprises a cylindrical main body portion and a blocking member-side threaded portion that is formed on an outer surface of the cylindrical main body portion and is screwable with the barrel-side threaded portion, and
an inner surface of the collar portion comprising the barrel-side threaded portion and an outer surface of the cylindrical main body portion comprising the blocking member-side threaded portion partially engage with each other at a time of screwing the barrel-side threaded portion and the blocking member-side threaded portion, and comprise a rotation restricting engagement portion that restricts rotation of the connection blocking member in the collar portion in a screwing release direction.

2. The syringe barrel according to claim 1, wherein the barrel-side threaded portion is a spiral recessed portion, and the blocking member-side threaded portion is a screw rib that is screwable with the spiral recessed portion.

3. The syringe barrel according to claim 2, wherein the connection blocking member comprises a protruding portion provided on an upper surface of the screw rib forming the blocking member-side threaded portion, the spiral recessed portion forming the barrel-side threaded portion comprises an inner surface site to be engaged with the protruding portion, and the rotation restricting engagement portion is formed by the protruding portion and the inner surface site of the spiral recessed portion.

4. The syringe barrel according to claim 3, wherein the inner surface site comprises a recessed portion that is engaged with the protruding portion and stores a vertex portion of the protruding portion.

5. The syringe barrel according to claim 2, wherein the screw rib forming the blocking member-side threaded portion comprises a raised portion formed higher than other portions, the spiral recessed portion forming the barrel-side threaded portion comprises an inner surface site to be engaged with the raised portion, and the rotation restricting engagement portion is formed by the raised portion and the inner surface site of the spiral recessed portion.

6. The syringe barrel according to claim 2, wherein the connection blocking member comprises the screw rib forming the blocking member-side threaded portion, the spiral recessed portion forming the barrel-side threaded portion comprises a low recessed site having a recessed portion depth shallower than other sites, and the rotation restricting engagement portion is formed by the low recessed site and an outer surface of the screw rib.

7. The syringe barrel according to any one of claims 1 to 6, wherein the syringe barrel comprises a distal end annular space formed between an inner surface of a cylindrical distal portion of the connection blocking member and an outer surface of the tip portion of the barrel.

8. The syringe barrel according to claim 3 or 5, wherein a protrusion amount and/or a width of the protruding portion or the raised portion decreases in a screwing direction of the screw rib of the connection blocking member.

9. The syringe barrel according to any one of claims 1 to 6, wherein the connection blocking member is formed of a thermoplastic resin harder than a thermoplastic resin forming the barrel.

10. The syringe barrel according to any one of claims 1 to 6, wherein the connection blocking member is formed of a thermoplastic resin having a thermal deformation temperature higher than a thermoplastic resin forming the barrel.

11. The syringe barrel according to any one of claims 2 to 6, wherein in the cylindrical main body portion of the connection blocking member, a portion where the screw rib is formed is a thick portion having a wall thickness larger than other sites.

12. The syringe barrel according to any one of claims 1 to 6, wherein the cylindrical main body portion of the connection blocking member comprises a cutout portion that opens in a distal end direction at a distal end side portion.

13. The syringe barrel according to any one of claims 1 to 6, wherein the syringe barrel comprises a cap that seals the tip portion, the barrel comprises a male screw portion provided on an outer surface of the collar portion, and the cap comprises a cap main body portion having a female screw portion screwed to the male screw portion of the collar portion, and a sealing member that is supported by the cap main body portion and abuts on a distal portion of the tip portion to seal the tip portion.

14. A syringe comprising: the syringe barrel according to any one of claims 1 to 6; a gasket slidably stored in the syringe barrel; and a plunger attached to or attachable to the gasket.

15. A drug preparation set comprising: the syringe barrel according to any one of claims 1 to 6; and
a connection medical tool comprising a connection portion connectable to the tip portion of the syringe barrel, wherein
the syringe barrel comprises a distal end annular space formed between an inner surface of a cylindrical distal portion of the connection blocking member and an outer surface of the tip portion of the barrel,
the connection portion of the connection medical tool comprises an inner tube portion capable of entering the distal end annular space of the syringe barrel and an outer tube portion covering the inner tube portion,
the inner tube portion has an inner surface shape that is liquid-tightly engageable with an outer surface of the tip portion, comprises an engagement portion that is engageable with an inner surface of the connection blocking member on an outer surface, and communicates with an inside of the barrel via the tip portion in a connection state between the tip portion of the barrel and the connection medical tool, and
the syringe barrel or the connection medical tool is rotated in a direction in which screwing between the barrel and the connection blocking member is released in a state in which the engagement portion of the inner tube portion of the connection medical tool and the inner surface of the connection blocking member are engaged, such that the connection blocking member is detached from the barrel together with the connection medical tool, and other medical tools are connectable to the tip portion of the barrel and/or the collar portion.

16. The drug preparation set according to claim 15, wherein an uneven structure engageable with each other is formed on an inner surface of the connection blocking member and an outer surface of the inner tube portion of the connection medical tool.

17. The drug preparation set according to claim 16, wherein the uneven structure comprises an annular protrusion extending in a circumferential direction.

18. The drug preparation set according to claim 16, wherein the uneven structure comprises a longitudinal rib extending in an axial direction.

19. The drug preparation set according to claim 16, wherein the uneven structure comprises a ratchet structure that rotates the connection blocking member only in a direction in which the connection blocking member is separated from the barrel.

20. The drug preparation set according to claim 15, wherein the connection medical tool is a vial adapter.

21. A method for manufacturing the syringe barrel according to claim 3, the method comprising:
a step of preparing the barrel and the connection blocking member;
a connection blocking member mounting step of disposing the connection blocking member between the tip portion and the collar portion by screwing, and engaging the protruding portion provided on an upper surface of the screw rib forming the blocking member-side threaded portion with a part of the spiral recessed portion forming the barrel-side threaded portion; and
a heating step of heating the barrel to which the connection blocking member is mounted by the connection blocking member mounting step, and embedding a vertex site of the protruding portion of the connection blocking member in an inner surface of the spiral recessed portion forming the barrel-side threaded portion by deformation of the barrel due to the heating.

22. The method for manufacturing the syringe barrel according to claim 21, wherein the heating step is performed by high-pressure steam sterilization.
